# EUROPÄISCHE PATENTSCHRIFT

(11) **EP 1 117 822 B1**
(45) Veröffentlichungstag und Bekanntmachung des Hinweises auf die Patenterteilung: **03.05.2006**
(21) Anmeldenummer: 99950660.3
(22) Anmeldetag: 07.10.1999
(51) Int. Cl.: C12P 19/18, C12N 9/10, C12N 15/54, C08B 30/00, A61K 47/36

(54) **HERSTELLUNG VON POLYGLUCANEN DURCH AMYLOSUCRASE IN GEGENWART EINER TRANSFERASE**
PREPARATION OF POLYGLUCANS BY AMYLOSUCRASE IN THE PRESENCE OF A TRANSFERASE
PREPARATION DES POLYGLUCANES PAR AMYLOSUCRASE EN PRESENCE D'UNE TRANSFERASE

(30) Priorität: 09.10.1998 DE 19846492
(43) Veröffentlichungstag der Anmeldung: 25.07.2001
(73) Patentinhaber: Südzucker AG Mannheim/Ochsenfurt, 68165 Mannheim (DE)
(72) Erfinder: GALLERT, Karl-Christian, D-61184 Karben (DE); BENGS, Holger, D-60598 Frankfurt am Main (DE); SIMANDI, Claus, D-65936 Frankfurt an Main (DE)
(74) Vertreter: Luderschmidt, Schüler & Partner GbR
(86) Internationale Anmeldenummer: PCT/EP1999/007518
(87) Internationale Veröffentlichungsnummer: WO 2000/022155

(56) Entgegenhaltungen:
- WO-A-00/14249
- WO-A-00/22140
- WO-A-95/31553
- OKADA, GENTARO ET AL: "New studies on amylosucrase, a bacterial.alpha.-D-glucosylase that directly converts sucrose to a glycogen-like.alpha.-glucan" J. BIOL. CHEM. (1974), 249(1), 126-35, XP000867741
- BUTTCHER, VOLKER ET AL: "Cloning and characterization of the gene for amylosucrase from Neisseria polysaccharea: production of a linear.alpha.-1,4-glucan" J. BACTERIOL. (1997), 179(10), 3324-3330, XP002129879
- DE MONTALK, G. POTOCKI ET AL: "Sequence analysis of the gene encoding amylosucrase from Neisseria polysaccharea and characterization of the recombinant enzyme" J. BACTERIOL. , 181(2), 375-381, Januar 1999 (1999-01), XP002138505

## Beschreibung

Die Erfindung hat zum Gegenstand die Herstellung von Polyglucan und Polyglucanderivaten mittels rekombinanter Amylosucrase in Gegenwart einer Transferase und/oder einer Glycosyltransferase.

Die biotechnologische Industrie ist interessiert an der Herstellung von neuen biologisch verträglichen Stoffen und Verfahren zu deren kostengünstigen Herstellung.

Die Herstellung von linearen Polyglucanen mittels biokatalytischer Herstellung ist beschrieben in WO 95/31553 unter Verwendung rekombinanter Amylosucrase. Im Rahmen dieser Erfindung wird sich ausdrücklich auf diese Schrift bezogen. Insbesondere zeigt die dort beschriebene rekombinante Amylosucrase die Aktivität der nativen Amylosucrase.

WO 95/31553 beschreibt ferner die Herstellung linearer Polysaccharide mittels Biotransformation, wobei das lineare Polysaccharid durch katalytische Reaktion von monomeren Grundbausteinen wie oligomeren Sacchariden, z.B. von Mono- und / oder Disacchariden hergestellt wird. Insbesondere wird Poly(1,4-alpha-D-glucan) in WO 95/31553 mittels einer Polysaccharidsynthase, die alpha-1,4-Glycosyltransferase oder synonym Amylosucrase (EC 2.4.1.4) synthetisiert. WO 95/31553 offenbart zudem Nukleinsäuresequenzen , welche in E. coli zu einem Protein mit der Aktivität einer Amylosucrase führt.

Die derart biokatalytisch erhaltenen linearen Polymere haben in vielen Anwendungen gegenüber verzweigten Polymeren einen Anwendungsvorteil hinsichtlich der Verarbeitbarkeit oder spezieller Eigenschaften, wie zum Beispiel die mechanische Stabilität und Beanspruchbarkeit. Darüber hinaus sind im pharmazeutischen (Human- und Veterinärbereich), medizinischen (Human- und Veterinärbereich), kosmetischen oder agrochemischen Bereich Anwendungen von großer kommerzieller Wichtigkeit, bei denen Eigenschaften oder Eigenschaftskombinationen benötigt werden, die durch lineare Polymere entweder nicht erhalten werden oder aber deren spezielle Eigenschaften für eine Anwendung in den oben genannten oder anderen Anwendungsbereichen über das für die spezielle Anwendung notwendige Maß hinausgehen. Das Erreichen spezieller Eigenschaften ist mit höheren Herstellungskosten verbunden. Sofern diese Eigenschaften in der Anwendung nicht benötigt werden, ist diese Vorgehensweise zu vermeiden. Eigenschaften, die nicht unbedingt die Verwendung linearer Polymere erfordern, können zum Beispiel in der besseren Formbarkeit der Herstellung von Probenkörpern spezieller Geometrie, der Porösität von Probenkörpern für die allgemeine Freisetzung von Wirkstoffen jedweder Art, insbesondere im Pharma- und Agrobereich, die Quellbarkeit, die leichtere chemische Modifizierbarkeit aufgrund leichter zugänglicher funktioneller Gruppen und anderes betreffen.

Okada et al. (J. Biol. Chem. (1974), 249, 126) beschreibt, daß in Gegenwart nativer Amylosucrase mit Verunreinigungen von Dextrinyltransferase zu spezifischen Verzweigungen im Molekül führt.

Bekannt ist ebenfalls, daß Primer die Aktivität nativer Amylosucrase beeinflussen (Vgl. DE 19860376.2).

Überraschender Weise zeigt sich, daß die Aktivität einer Amylosucrase in

Gegenwart einer Transferase/Glycosyltansferase nicht beeinträchtigt, sondern vielmehr im Rahmen der Produktion vorteilhaft gesteigert ist.

Für die industrielle Produktion ist es von Bedeutung, wirtschaftlich wertvolle Produkte zu erhalten. Zudem sollen Produkte erhalten werden, die biokompatibel sind und für zahlreiche biowissenschaftliche und materialwissenschaftliche Anwendungen verwendet werden können. Der Vorteil solcher Produkte liegt darin, daß sie unter anderem für den Einsatz an und in Lebewesen, besonders im Humanbereich geeignet sind (Kosmetik, Lebensmittel, Pharmazie, Medizin) und durch die Biokompatibilität ebenfalls die Entsorgung nach ihrem Einsatz in technischen Bereichen überwiegend problemlos ist.

Daher ist es Aufgabe der Erfindung rekombinante Amylosucrase modifiziert in biotechnologischen Verfahren zur Polyglucan - Herstellung und deren Derivate in vitro einzusetzen und neue Produkte zu erhalten. In vitro Verfahren ermöglichen die Herstellung reproduzierbarer Produkte gleicher Qualität und Güte (siehe Beispiel).

Die erfindungsgemäße Aufgabe wird dadurch gelöst, daß rekombinante Polyglucansucrase in Gegenwart einer Transferase/Glycosyltransferase eingesetzt und zur Herstellung von Polyglucan und Polyglucanderivaten verwendet wird.

Besonders bevorzugt sind solche Polyglucansucrasen, wie die mit SEQ. ID. NO.1 Daher betrifft die Erfindung eine Amylosucrase mit der Aminosäuresequenz SEQ ID No. 1 oder eine redundante Variante.

Im Sinne dieser Erfindung umfaßt der Begriff Polyglucan und Polyglucanderivate insbesondere Amylose und Amylosederivate.

Vorteilhaft ist die überraschend hohe Produkteinheitlichkeit der erhaltenen Polyglucan und Polyglucanderivate, insbesondere der erzielten Molekulargewichte. Je nach der Verwendung einzelner biogener Stoffe, insbesondere Enzyme, können sehr unterschiedliche Polyglucane und /oder Polyglucanderivate erhalten werden, deren Molekulargewicht von 10³ bis 10⁹ Dalton variieren. Bevorzugte Molekulargewichte liegen im Bereich von 5 x von 10³ bis 10⁶ Dalton, besonders bevorzugt im Bereich von 5 x von 10³ bis 5 x von 10⁴ Dalton.

Vorteilhaft ist die Vielfalt der erhaltenen Produkte und deren möglichen Kombination. Hieraus, insbesondere bei geeigneter Kombination der Polymere hinsichtlich ihrer Molekulargewichte und / oder zugrundeliegenden Primärstrukturen können besondere Eigenschaftsmerkmale kombiniert werden oder aber die Verarbeitbarkeit in spezieller Weise beeinflußt werden. Dies gilt insbesondere bei der Verarbeitung nach Verarbeitungsverfahren der klassischen Polymerchemie, insbesondere in technischen Anwendungen.

Die Polyglucane und Polyglucanderivate zeichnen sich darüber hinaus durch eine hohe Vielfalt aus, die durch die Polydispersität der Polyglucane und Polyglucanderivate bestimmt werden. Die Polydispersität kann dabei in weiten Bereichen variieren. Dabei sind für verschiedene Verwendungen durchaus verschiedene Polydispersitäten von Interesse. Die Polydispersität, die sich aus dem Quotienten von Polymergewichtsmittelwert und Polymerzahlenmittelwert ergibt, kann von 1,0 bis 100 oder größer variieren, wobei für spezielle Anwendungen Polydispersitäten im Bereich von 1,1 bis 15 bevorzugt sind. Besonders vorteilhaft zeichnen sich Polyglucane oder Polyglucanderivate aus, die Polydispersitäten im Bereich von 1,1 bis 5 aufweisen.

"Biokompatibel" bedeutet, daß die eingesetzten Polysaccharide einem vollständigen biologischen Abbau unterzogen werden und keine schädliche Anreicherung in der Nahrungskette insbesondere dem humanen Organismus erfolgt.

Unter biologischen Abbau ist dabei jedweder in vivo ablaufende Vorgang angesprochen, der zu einem Abbau oder Zerstörung des Polymers führt. Insbesondere fallen ebenfalls hydrolytische oder enzymatische Prozesse in diesen Bereich. Für die Biokompatibilität der Polysaccharide sowie seiner Abbauprodukte (Metabolite) ist nicht zuletzt auch der naturidentische Charakter der eingesetzten Polysaccharide von hoher Bedeutung. Daher sind die in Frage kommenden Polysaccharide ebenfalls für den therapeutischen, diagnostischen oder prophylaktischen Einsatz besonders geeignet.

Insbesondere können durch sogenannte Enzymgemische gesteigerte Ausbeuten an Polyglucan und Polyglucanderivate erhalten werden.

Als vorteilhafte Enzyme kommen vorzugsweise in Betracht ohne, daß die nachfolgende Liste einen abschließenden Charakter hat: Transferasen und Glycosyltransferasen
- 2.4:1.1: Phosphorylase.
- 2.4.1.2: Dextrin dextranase.
- 2.4.1.5: Dextransucrase.
- 2.4.1.7: Sucrose phosphorylase.
- 2.4.1.8: Maltose phosphorylase.
- 2.4.1.9: Inulosucrase.
- 2.4.1.10: Levansucrase.
- 2.4.1.11: Glycogen (starch) synthase.
- 2.4.1.12: Cellulose synthase (UDP-forming).
- 2.4.1.13: Sucrose synthase.
- 2.4.1.14: Sucrose-phosphate synthase.
- 2.4.1.15: Alpha,alpha-trehalose-phosphate synthase (UDP-forming).
- 2.4.1.16: Chitin synthase.
- 2.4.1.17: UDP-glucuranosyltransferase.
- 2.4.1.18: 1,4-alpha-glucan branching enzyme.
- 2.4.1.19: Cyclomaltodextrin glucanotransferase.
- 2.4.1.20: Cellobiose phosphorylase.
- 2.4.1.21: Starch (bacterial glycogen) synthase.
- 2.4.1.22: Lactose synthase.
- 2.4.1.23: Sphingosine beta-galactosyltransferase.
- 2.4.1.24: 1,4-alpha-glucan 6-alpha-glucosyltransferase.
- 2.4.1.25: 4-alpha-glucanotransferase.
- 2.4.1.26: DNA alpha-glucosyltransferase.
- 2.4.1.27: DNA beta-glucosyltransferase.
- 2.4.1.28: Glucosyl-DNA beta-glucosyltransferase.
- 2.4.1.29: Cellulose synthase (GDP-forming).
- 2.4.1.30: 1,3-beta-oligoglucan phosphorylase.
- 2.4.1.31: Laminaribiose phosphorylase.
- 2.4.1.32: Glucomannan 4-beta-mannosyltransferase.
- 2.4.1.33: Alginate synthase.
- 2.4.1.34: 1,3-beta-glucan synthase.
- 2.4.1.35: Phenol beta-glucosyltransferase.
- 2.4.1.36: Alpha,alpha-trehalose-phosphate synthase (GDP-forming).
- 2.4.1.37: Glycoprotein-fucosylgalactoside alpha-galactosyltransferase.
- 2.4.1.38: Beta-N-acetylglucosaminyl-glycopeptide beta-1,4-galactosyltransferase.
- 2.4.1.39: Steroid N-acetylglucosaminyltransferase.
- 2.4.1.40: Glycoprotein-fucosylgalactoside alpha-N-acetylgalactosaminyltransferase.
- 2.4.1.41: Polypeptide N-acetylgalactosaminyltransferase.
- 2.4.1.43: Polygalacturonate 4-alpha-galacturonosyltransferase.
- 2.4.1.44: Lipopolysaccharide galactosyltransferase.
- 2.4.1.45: 2-hydroxyacylsphingosine 1-beta-galactosyltransferase.
- 2.4.1.46: 1,2-diacylglycerol 3-beta-galactosyltransferase.
- 2.4.1.47: N-acylsphingosine galactosyltransferase.
- 2.4.1.48: Heteroglycan alpha-mannosyltransferase.
- 2.4.1.49: Cellodextrin phosphorylase.
- 2.4.1.50: Procollagen galactosyltransferase.
- 2.4.1.52: Poly(glycerol-phosphate) alpha-glucosyltransferase.
- 2.4.1.53: Poly(ribitol-phosphate) beta-glucosyltransferase.
- 2.4.1.54: Undecaprenyl-phosphate mannosyltransferase.
- 2.4.1.55: Transferred entry: 2.7.8.14.
- 2.4.1.56: Lipopolysaccharide N-acetylglucosaminyltransferase.
- 2.4.1.57: Phosphatidyl-myo-inositol alpha-mannosyltransferase.
- 2.4.1.58: Lipopolysaccharide glucosyltransferase I.
- 2.4.1.60: Abequosyltransferase.
- 2.4.1.62: Ganglioside galactosyltransferase.
- 2.4.1.63: Linamarin synthase.
- 2.4.1.64: Alpha,alpha-trehalose phosphorylase.
- 2.4.1.65: Galactoside 3(4)-L-fucosyltransferase.
- 2.4.1.66: Procollagen glucosyltransferase.
- 2.4.1.67: Galactinol-raffinose galactosyltransferase.
- 2.4.1.68: Glycoprotein 6-alpha-L-fucosyltransferase.
- 2.4.1.69: Galactoside 2-L-fucosyltransferase.
- 2.4.1.70: Poly(ribitol-phosphate) N-acetylglucosaminyltransferase.
- 2.4.1.71: Arylamine glucosyltransferase.
- 2.4.1.72: Transferred entry: 2.4.2.24.
- 2.4.1.73: Lipopolysaccharide glucosyltransferase II.
- 2.4.1.74: Glycosaminoglycan galactosyltransferase.
- 2.4.1.75: UDP-galacturonosyltransferase.
- 2.4.1.78: Phosphopolyprenol glucosyltransferase.
- 2.4.1.79: Galactosylgalactosylglucosylceramide beta-D-acetylgalactosaminyltransferase.
- 2.4.1.80: Ceramide glucosyltransferase.
- 2.4.1.81: Flavone 7-O-beta-glucosyltransferase.
- 2.4.1.82: Galactinol-sucrose galactosyltransferase.
- 2.4.1.83: Dolichyl-phosphate beta-D-mannosyltransferase.
- 2.4.1.85: Cyanohydrin beta-glucosyltransferase.
- 2.4.1.86: Glucosaminylgalactosylglucosylceramide beta-galactosyltransferase.
- 2.4.1.87: Beta-galactosyl-N-acetylglucosaminylglycopeptide alpha-1,3-galactosyltransferase.
- 2.4.1.88: Globoside alpha-N-acetylgalactosaminyltransferase.
- 2.4.1.90: N-acetyllactosamine synthase.
- 2.4.1.91: Flavonol 3-O-glucosyltransferase.
- 2.4.1.92: (N-acetylneuraminyl)-galactosylglucosylceramide N-acetylgalactosaminyltransferase.
- 2.4.1.93: Inulin fructotransferase (depolymerizing).
- 2.4.1.94: Protein N-acetylglucosaminyltransferase.
- 2.4.1.95: Bilirubin-glucuronoside glucuronosyltransferase.
- 2.4.1.96: Sn-glycerol-3-phosphate 1-galactosyltransferase.
- 2.4.1.97: 1,3-beta-glucan phosphorylase.
- 2.4.1.99: Sucrose 1 F-fructosyltransferase.
- 2.4.1.100: 1,2-beta-fructan 1 F-fructosyltransferase.
- 2.4.1.101: Alpha-1,3-mannosyl-glycoprotein beta-1,2-N-acetylglucosaminyltransferase.
- 2.4.1.102: Beta-1,3-galactosyl-O-glycosyl-glycoprotein beta-1,6-N-acetylglucosaminyltransferase.
- 2.4.1.103: Alizarin 2-beta-glucosyltransferase.
- 2.4.1.104: O-dihydroxycoumarin 7-O-glucosyltransferase.
- 2.4.1.105: Vitexin beta-glucosyltransferase.
- 2.4.1.106: Isovitexin beta-glucosyltransferase.
- 2.4.1.109: Dolichyl-phosphate-mannose-protein mannosyltransferase.
- 2.4.1.110: tRNA-queuosine beta-mannosyltransferase.
- 2.4.1.111: Coniferyl-alcohol glucosyltransferase.
- 2.4.1.112: Alpha-1,4-glucan-protein synthase (UDP-forming).
- 2.4.1.113: Alpha-1,4-glucan-protein synthase (ADP-forming).
- 2.4.1.114: 2-coumarate O-beta-glucosyltransferase.
- 2.4.1.115: Anthocyanidin 3-O-glucosyltransferase.
- 2.4.1.116: Cyanidin-3-rhamnosylglucoside 5-O-glucosyltransferase.
- 2.4.1.117: Dolichyl-phosphate beta-glucosyltransferase.
- 2.4.1.118: Cytokinin 7-beta-glucosyltransferase.
- 2.4.1.119: Dolichyl-diphosphooligosaccharide-protein glycosyltransferase.
- 2.4.1.120: Sinapate 1-glucosyltransferase.
- 2.4.1.121: Indole-3-acetate beta-glucosyltransferase.
- 2.4.1.122: Glycoprotein-N-acetylgalactosamine 3-beta-galactosyltransferase.
- 2.4.1.123: Inositol 1-alpha-galactosyltransferase.
- 2.4.1.124: N-acetyllactosamine 3-alpha-galactosyltransferase.
- 2.4.1.125: Sucrose-1,6-alpha-glucan 3(6)-alpha-glucosyltransferase.
- 2.4.1.126: Hydroxycinnamate 4-beta-glucosyltransferase.
- 2.4.1.127: Monoterpenol beta-glucosyltransferase.
- 2.4.1.128: Scopoletin glucosyltransferase.
- 2.4.1.129: Peptidoglycan glycosyltransferase.
- 2.4.1.130: Dolichyl-phosphate-mannose-glycolipid alpha-mannosyltransferase.
- 2.4.1.131: Glycolipid 2-alpha-mannosyltransferase.
- 2.4.1.132: Glycolipid 3-alpha-mannosyltransferase.
- 2.4.1.133: Xylosylprotein 4-beta-galactosyltransferase.
- 2.4.1.134: Galactosylxylosylprotein 3-beta-galactosyltransferase.
- 2.4.1.135: Galactosylgalactosylxylosylprotein 3-beta-glucuronosyltransferase.
- 2.4.1.136: Gallate 1-beta-glucosyltransferase.
- 2.4.1.137: Sn-glycerol-3-phosphate 2-alpha-galactosyltransferase.
- 2.4.1.138: Mannotetraose 2-alpha-N-acetylglucosaminyltransferase.
- 2.4.1.139: Maltose synthase.
- 2.4.1.140: Altemansucrase.
- 2.4.1.141: N-acetylglucosaminyldiphosphodolichol N-acetylglucosaminyltransferase.
- 2.4.1.142: Chitobiosyldiphosphodolichol alpha-mannosyltransferase.
- 2.4.1.143: Alpha-1,6-mannosyl-glycoprotein beta-1,2-N-acetylglucosaminyltransferase.
- 2.4.1.144: Beta-1,4-mannosyl-glycoprotein beta-1,4-N-acetylglucosaminyltransferase.
- 2.4.1.145: Alpha-1,3-mannosyl-glycoprotein beta-1,4-N-acetylglucosaminyltransferase.
- 2.4.1.146: Beta-1,3-galactosyl-O-glycosyl-glycoprotein beta-1,3-N-acetylglucosaminyltransferase.
- 2.4.1.147: Acetylgalactosaminyl-O-glycosyl-glycoprotein beta-1,3-N-acetylglucosaminyltransferase.
- 2.4.1.148: Acetylgalactosaminyl-O-glycosyl-glycoprotein beta-1,6-N-acetylglucosaminyltransferase.
- 2.4.1.149: N-acetyllactosaminide beta-1,3-N-acetylglucosaminyltransferase.
- 2.4.1.150: N-acetyllactosaminide beta-1,6-N-acetylglucosaminyltransferase.
- 2.4.1.151: N-acetyllactosaminide alpha-1,3-galactosyltransferase.
- 2.4.1.152: Galactoside 3-fucosyltransferase.
- 2.4.1.153: Dolichyl-phosphate alpha-N-acetylglucosaminyltransferase.
- 2.4.1.154: Globotriosylceramide beta-1,6-N-acetylgalactosaminyltransferase.
- 2.4.1.155: Alpha-1,3(6)-mannosylglycoprotein beta-1,6-N-acetylglucosaminyltransferase.
- 2.4.1.156: Indolylacetyl-myo-inositol galactosyltransferase.
- 2.4.1.157: 1,2-diacylglycerol 3-glucosyltransferase.
- 2.4.1.158: 13-hydroxydocosanoate 13-beta-glucosyltransferase.
- 2.4.1.159: Flavonol-3-O-glucoside L-rhamnosyltransferase.
- 2.4.1.160: Pyridoxine 5'-O-beta-D-glucosyltransferase.
- 2.4.1.161: Oligosaccharide 4-alpha-D-glucosyltransferase.
- 2.4.1.162: Aldose beta-D-fructosyltransferase.
- 2.4.1.163: Beta-galactosyl-N-acetylglucosaminylgalactosyl-glucosylceramide Beta-1,3-acetylglucosaminyltransferase.
- 2.4.1.164: Galactosyl-N-acetylglucosaminylgalactosyl-glucosylceramide beta-1,6-N-acetylglucosaminyltransferase.
- 2.4.1.165: N-acetylneuraminylgalactosylglucosylceramide beta-1,4-N-acetylgalactosaminyltransferase.
- 2.4.1.166: Raffinose-raffinose alpha-galactosyltransferase.
- 2.4.1.167: Sucrose 6(F)-alpha-galactosyltransferase.
- 2.4.1.168: Xyloglucan 4-glucosyltransferase.
- 2.4.1.169: Xyloglucan 6-xylosyltransferase.
- 2.4.1.170: lsoflavone 7-O-glucosyltransferase.
- 2.4.1.171: Methyl-ONN-azoxymethanol glucosyltransferase.
- 2.4.1.172: Salicyl-alcohol glucosyltransferase.
- 2.4.1.173: Sterol glucosyltransferase.
- 2.4.1.174: Glucuronylgalactosylproteoglycan beta-1,4-N-acetylgalactosaminyltransferase.
- 2.4.1.175: Glucuronyl-N-acetylgalactosaminylproteoglycan beta-1,4-N-acetylgalactosaminyltransferase.
- 2.4.1.176: Gibberellin beta-glucosyltransferase.
- 2.4.1.177: Cinnamate glucosyltransferase.
- 2.4.1.178: Hydroxymandelonitrile glucosyltransferase.
- 2.4.1.179: Lactosylceramide beta-1,3-galactosyltransferase.
- 2.4.1.180: Lipopolysaccharide N-acetylmannosaminouronosyltransferase.
- 2.4.1.181: Hydroxyanthraquinone glucosyltransferase.
- 2.4.1.182: Lipid-A-disaccharide synthase.
- 2.4.1.183: Alpha-1,3-glucan synthase.
- 2.4.1.184: Galactolipid galactosyltransferase.
- 2.4.1.185: Flavonone 7-O-beta-glucosyltransferase.
- 2.4.1.186: Glycogenin glucosyltransferase.
- 2.4.1.187: N-acetylglucosaminyldiphosphoundecaprenol N-acetyl-beta-D-mannosaminyltransferase.
- 2.4.1.188: N-acetylglucosaminyldiphosphoundecaprenol glucosyltransferase.
- 2.4.1.189: Luteolin 7-O-glucoronosyltransferase.
- 2.4.1.190: Luteolin-7-O-glucuronide 7-O-glucuronosyltransferase.
- 2.4.1.191: Luteolin-7-O-diglucuronide 4'-O-glucuronosyl-transferase.
- 2.4.1.192: Nuatigenin 3-beta-glucosyltransferase.
- 2.4.1.193: Sarsapogenin 3-beta-glucosyltransferase.
- 2.4.1.194: 4-hydroxybenzoate 4-O-beta-D-glucosyltransferase.
- 2.4.1.195: Thiohydroximate beta-D-glucosyltransferase.
- 2.4.1.196: Nicotinate glucosyltransferase.
- 2.4.1.197: High-mannose-oligosaccharide beta-1,4-N-acetylglucosaminyltransferase.
- 2.4.1.198: Phosphatidylinositol N-acetylglucosaminyltransferase.
- 2.4.1.199: Beta-mannosylphosphodecaprenol-mannooligosaccharide 6-mannosyltransferase.
- 2.4.1.200: Inulin fructotransferase (depolymerizing, difructofuranose-1,2':2',1-dianhydride-forming).
- 2.4.1.201: Mannosyl-glycoprotein beta-1,4-N-acetylglucosaminyl-transferase.
- 2.4.1.202: 2,4-dihydroxy-7-methoxy-2H-1,4-benzoxazin-3(4H)-one 2-D-glucosyltransferase.
- 2.4.1.203: Zeatin O-beta-D-glucosyltransferase.
- 2.4.1.204: Zeatin O-beta-D-xylosyltransferase.
- 2.4.1.205: Galactogen 6-beta-galactosyltransferase.
- 2.4.1.206: Lactosylceramide 1,3-N-acetyl-beta-D-glucosaminyl-transferase.
- 2.4.1.207: Xyloglucan:xyloglucosyl transferase.
- 2.4.1.208: Diglucosyl diacylglycerol (DGlcDAG) synthase.
- 2.4.2.1: Purine-nucleoside phosphorylase.
- 2.4.2.2: Pyrimidine-nucleoside phosphorylase.
- 2.4.2.3: Uridine phosphorylase.
- 2.4.2.4: Thymidine phosphorylase.
- 2.4.2.5: Nucleoside ribosyltransferase.
- 2.4.2.6: Nucleoside deoxyribosyltransferase.
- 2.4.2.7: Adenine phosphoribosyltransferase.
- 2.4.2.8: Hypoxanthine phosphoribosyltransferase.
- 2.4.2.9: Uracil phosphoribosyltransferase.
- 2.4.2.10: Orotate phosphoribosyltransferase.
- 2.4.2.11: Nicotinate phosphoribosyltransferase.
- 2.4.2.12: Nicotinamide phosphoribosyltransferase.
- 2.4.2.13: Transferred entry: 2.5.1.6.
- 2.4.2.14: Amidophosphoribosyltransferase.
- 2.4.2.15: Guanosine phosphorylase.
- 2.4.2.16: Urate-ribonucleotide phosphorylase.
- 2.4.2.17: ATP phosphoribosyltransferase.
- 2.4.2.18: Anthranilate phosphoribosyltransferase.
- 2.4.2.19: Nicotinate-nucleotide pyrophosphorylase (carboxylating).
- 2.4.2.20: Dioxotetrahydropyrimidine phosphoribosyltransferase.
- 2.4.2.21: Nicotinate-nucleotide-dimethylbenzimidazole phosphoribosyltransferase.
- 2.4.2.22: Xanthine-guanine phosphoribosyltransferase.
- 2.4.2.23: Deoxyuridine phosphorylase.
- 2.4.2.24: 1,4-beta-D-xylan synthase.
- 2.4.2.25: Flavone apiosyltransferase.
- 2.4.2.26: Protein xylosyltransferase.
- 2.4.2.27: dTDP-dihydrostreptose-streptidine-6-phosphate dihydrostreptosyltransferase.
- 2.4.2.28: 5'-methylthioadenosine phosphorylase.
- 2.4.2.29: Queuine tRNA-ribosyltransferase.
- 2.4.2.30: NAD(+) ADP-ribosyltransferase.
- 2.4.2.31: NAD(P)(+)-arginine ADP-ribosyltransferase.
- 2.4.2.32: Dolichyl-phosphate D-xylosyltransferase.
- 2.4.2.33: Dolichyl-xylosyl-phosphate-protein xylosyltransferase.
- 2.4.2.34: Indolylacetylinositol arabinosyltransferase.
- 2.4.2.35: Flavonol-3-O-glycoside xylosyltransferase.
- 2.4.2.36: NAD(+)-diphthamide ADP-ribosyltransferase.
- 2.4.2.37: NAD(+)-dinitrogen-reductase ADP-D-ribosyltransferase.
- 2.4.99.1: Beta-galactosamide alpha-2,6-sialyltransferase.
- 2.4.99.2: Monosialoganglioside sialyltransferase.
- 2.4.99.3: Alpha-N-acetylgalactosaminide alpha-2,6-sialyltransferase.
- 2.4.99.4: Beta-galactoside alpha-2,3-sialyltransferase.
- 2.4.99.5: Galactosyldiacylglycerol alpha-2,3-sialyltransferase.
- 2.4.99.6: N-acetyllactosaminide alpha-2,3-sialyltransferase.
- 2.4.99.7: (Alpha-N-acetyl-neuraminyl-2,3-beta-galactosyl-1,3)-N-acetylgalactosaminide alpha-2,6-sialyltransferase.
- 2.4.99.8: Alpha-N-acetyl-neuraminide alpha-2,8-sialyltransferase.
- 2.4.99.9: Lactosylceramide alpha-2,3-sialyltransferase.
- 2.4.99.10: Neolactotetraosylceramide alpha-2,3-sialyltransferase.
- 2.4.99.11: Lactosylceramide alpha-2,6-N-sialyltransferase.

Ein Gegenstand der Erfindung ist daher die kostengünstige Derivatisierung von Polyglucan.

Die Derivatisierung bedeutet im Sinne dieser Erfindung, daß die natürlicherweise im Polyglucan vorkommenden funktionellen Gruppen, Hydroxylgruppen (auch: Alkoholfunktionen), derivatisiert, ersetzt, modifiziert oder chemisch substituiert sind. Unter Derivatisierung wird daher ebenfalls das Einführen von Verzweigungen mittels biogener Stoffe, insbesondere genannter Enzyme verstanden.

Derivate sind ebenfalls jene, die eine Umsetzung spezifisch an einem der C-Atome C-2, C-3 oder C-6 erfahren und zwar von> 0 % bis maximal 100 %, oder das Mischungen auftreten, d.h. unterschiedliche prozentuale Derivatisierungen an unterschiedlichen Positionen im C6 Körper einer Glucaneinheit. Im Fall der Hydroxylgruppe am C-6 Atom des Glucangrundkörpers im Polyglucan können insbesondere vorteilhafterweise Verzweigungsgrade von 1 % bis 40 % erhalten werden. Insbesondere zeichnen sich diese Polyglucanderivate durch Verzweigungsgrade von 2 % bis 10 % aus. Insbesondere zeichnen sich die Art der Verzweigungen und die Anzahl der Verzweigungen in den beschriebenen Polyglucanderivaten dadurch aus, daß sie sich von denen der natürlichen, das heißt in der Natur vorkommenden Polyglucane, wie sie aus Pflanzen, Tieren oder anderen Organismen gewonnen werden können, unterscheiden können.

Ein weiterer Gegenstand dieser Erfindung ist daher ebenfalls die modifizierte Herstellung von PolyglucanDies bedeutet im Sinne dieser Erfindung die Zugabe von Transferase/Glycosyltransferase, bereits während der Expression der Amylosucrase mit anschließender Biotransformation und Synthese der Polyglucane oder Polyglucanderivaten und/oder die Nachbehandlung mit Transferase/Glycosyltransferase solcher hergestellten Produkte.

Insofern können mittels der dargelegten Erfindung folgende Derivate erhalten werden, ohne das die Aufzählung abschließend ist
A) Polyglucan-Ester
   acetate
   nitrate
   phosphate
   xanthogenate
   citrate
B) Polyglucan-Ether
   hydroxyethyl
   hydroxypropyl
   1,2-dihydroxypropyl
   carboxymethyl
C) Polyglucan-Abbauprodukte durch Oxidation oder partielle Ringöffnung
   Dialdehydamylose
   Carboxyamylose
   Persulfat abgebautes Polyglucan
D) Natürliche Polymere (naturidentische Polymere / Polyglucane)
   Amylopektin
   Glykogen
   und/oder Pfropfpolymere, Blockpolymere, Copoloymere, statistische Copolymere, alternierende Copolymere und dendritische Copolymere, einschließlich Stern- und Leiterpolymere sowie Bandpolymere.

Der Begriff Copolymer umfaßt Polyglucan und / oder Polyglucanderivate aus 2 oder mehr Grundeinheiten (Monomere).

Für den Erfindungsgegenstand ist es maßgebend entweder die genannte Biotransformation in modifizierter Form durchzuführen oder das erhaltene Polyglucanprodukt nach erfolgter Polymerisation in einer zweiten Reaktion, bevorzugt einer Biokatalyse, zu verändern. Dies geschieht dadurch, daß das Polyglucan oder Polyglucanderivat möglichst weitgehendst von allen Reaktionspartner / Parametern der Biotransformation isoliert und in einer weiteren Reaktion weiter verändert wird. Dies kann durch die Verwendung des Zusatzes von weiteren biogenen Verbindungen erfolgen, vorzugsweise Enzymen.

Eine andere Einteilung der verschiedenen Reaktionswege zur Herstellung von Polyglucanen und / oder Polyglucanderivaten kann folgendermaßen beschrieben werden.

Die Modifikation der Polyglucane oder Polyglucanderivate kann darüber erfolgen, ob nur der Reaktionsverlauf der Biotransformation, also der Umsetzung von Saccharose und seinen Derivaten zu Polyglucan und / oder Polyglucanderivaten verändert werden soll (Dies kann zum Beispiel durch den Verzehr der bei der

Biotransformation gebildeten Fruktose geschehen), oder es kann durch die Reaktion oder Reaktionsführung bei der Umsetzung von Amylosucrase mit Transferase/Glycosiltransferase direkt ein Polyglucan und / oder ein oder mehrere Polyglucanderivate gebildet werden. Dabei schließt das eine das andere nicht aus. Als Beispiel ist hier der veränderte Reaktionsverlauf zu nennen, der dadurch entsteht, daß man durch Phosphorylierung und / oder Methylierung und / oder Sulfatierung und / oder weitere Modifikationen eine erhöhte oder eine emiedrigte Löslichkeit des Polyglucans erhält und auf diese Weise zum Beispiel veränderte Kettenlängen o.ä. erhält.

Des weiteren können weitere modifizierende Enzyme z.B. im gleichen Operon wie die Amylosucrase kodiert sein, und mit diesem Enzym parallel gereinigt werden. Auf diese Weise entsteht bereits bei der Herstellung und Separation der Amylosucrase ein Mix von verschiedenen biogenen Stoffen, vorzugsweise verschiedenen Enzymen inklusive Amylosucrase, die in einer Biokatalyse (Biotransformation) eingesetzt werden können.

Dem Fachmann sind derartige Mischungen auch unter dem Begriff "Polymerblends" bekannt. Beispielsweise könnten derartige modifizierte Amylosucrasen bewirken, daß in ihrer chemischen Struktur variierte Zucker, Monosaccharide, Disaccharide, Oligosaccharide, besser in die Polymerstruktur eingebunden werden können, im Sinne einer schnelleren Reaktion oder im Sinne einer höheren Verträglichkeit mit anderen monomeren Zuckereinheiten, die in das Polymerrückgrat eingebaut werden. Aber auch ein Kettenabbruch, der zu speziellen funktionellen Molekülen aufgrund ihrer speziellen Polymerendgruppen führen können, die besondere Eigenschaften aufweisen, z.B. oberflächenaktives Verhalten, im weitesten Sinne von amphiphilen Molekülen, führt, ist nicht auszuschließen und kann sogar zur bevorzugten Reaktion werden.

Die enzymatisch, durch den Einsatz von Tranferase/Glycosyltransferase modifizierten Polyglucane können als Ausgangsmaterial für weitere, chemische Modifikationen eingesetzt werden.

Die erhaltenen Polyglucane und/oder Polyglucanderivate können, wie folgt, verwendet werden Verwendung als pharmazeutische und / oder agrochemische Formulierung zur Ausbringung in der Landwirtschaft (wie Tablette, Kapselinhattsstoff, Suspensionen, Emulsionen und andere dem Durchschnittsfachmann auf den genannten Gebieten bekannten Formulierungen), Wirkstoffträger und/oder Depotformulierung, insbesondere Tablettenhilfsstoff, Verwendung als Lebensmittel und / oder Lebensmittelzusatzstoff, Verwendung als kosmetischer Zusatzstoff. Diese vorteilhaften Verwendungen der Polyglucane sind begründet in der Wahrung der Biokompatibilität, durch die erfindungsgemäße Verwendung von Transferase/Glycosyltransferase.

Wirkstoffe sind Verbindungen, die für den biologischen Organismus, insbesondere Mensch, Tier, Pflanze, einen pallativen oder kurativen Effekt aufweisen. Dabei fallen unter den Begriff Wirkstoff im allgemeinen Sinn auch agrochemische Verbindungen, mit fungizider, pestizider, insektizider, herbizider Wirkung, jedoch auch allgemein solche Verbindungen, die einen nützlichen Effekt in der Land-, Forst- oder Gartenwirtschaft aufweisen, zum Beispiel Düngemittel. Auch Duft- oder Aromastoffe, die insbesondere im Lebensmittelbereich oder der Kosmetik Anwendungen finden, fallen unter den Begriff Wirkstoff. Insofern werden ausdrücklich alle Wirkstoffe mit einbezogen, die einen therapeutischen und / oder prophylaktischen und / oder dekorativen Effekt aufweisen.

### Beispiele

Beschreibung der wichtigsten Sequenzen:
SEQ ID No. 1 beschreibt eine Aminosäuresequenz mit der Aktivität einer Amylosucrase erhältlich mittels Rekombinationstechnologie in E. coli aus einer DNA des Organismus Neisseira polysaccharea und wie in WO 95/31553 gezeigt.

### Beispiel 1:

Die Mischung von Amylosucrase und einem weiteren Enzym einer anderen Aktivität führt zu einem derivatisiertem Amyloseprodukt, wie folgt:. Ein Beispiel ist hier die Mischung von Amylosucrase mit der Amylo-1,4→1,6-Transglycosylase. Dieses Enzym katalysiert die Einführung von 1,6-Verzweigungen in linearen Amylose-Molekülen mit einer Mindestlänge von 6-11 Glucoseeinheiten. Dieser Größenbereich liegt genau im Bereich der von der Amylosucrase hergestellten Glucose-Polymere. Es entsteht somit ein stark verzweigtes aber sehr kurzkettiges Molekül.
Zur Durchführung des Experiments werden in einem 10 ml Volumen mit 50 mM NaCitrat-Puffer mit einem pH-Wert von 6.5, 2 g D-Glucose und 0,02% NaN3 mit 200 U rekombinant hergestellter Amylosucrase versetzt. Zusätzlich werden 10 U der Amylo-1,4→1,6-Trans-glycosylase in den Ansatz gegeben. Der Ansatz wird bei 37°C ohne Durchmischung für 72 h Inkubiert. Die entstehenden Produkte werden mit Ethanol gefällt und über GPC analysiert. Unter den genannten Bedingungen entstehen etwa 0,75 g Polymeres Produkt mit einem Verzweigungsgrad von 10 %.

## Patentansprüche

1. Verfahren zur Herstellung von Polyglucanen und / oder Polyglucanderivaten, **dadurch gekennzeichnet, dass** Amylosucrase in vitro mit mindestens einer Transferase und / oder einer Glycosyltransferase versetzt wird.

2. Verfahren nach Anspruch 1, **dadurch gekennzeichnet, dass** die Amylosucrase eine Aminosäuresequenz gemäß SEQ ID. No. 1 umfasst.

3. Verfahren nach Anspruch 1, **dadurch gekennzeichnet, dass** das Polyglucanderivat ein Polyglucan-Ester oder ein Polyglucan-Ether ist.

4. Verfahren nach Anspruch 1, **dadurch gekennzeichnet, dass** das Polyglucanderivat ein Propf-, Block-, Co-, statistisches Copolymer oder ein Stern-, Leiter- oder Bandpolymer ist.

5. Verfahren zur Herstellung eines Medikamentes, **dadurch gekennzeichnet, dass** mindestens ein nach einem der Ansprüche 1 bis 4 hergestelltes Polyglucan und / oder Polyglucanderivat als Wirkstoffträger verwendet wird.

6. Verfahren zur Herstellung eines Medikamentes, **dadurch gekennzeichnet, dass** mindestens ein nach einem der Ansprüche 1 bis 4 hergestelltes Polyglucan und / oder Polyglucanderivat als Depotsystem für mindestens einen Wirkstoff mit einem therapeutischen oder prophylaktischem Effekt verwendet wird.

7. Verfahren zur Herstellung eines Medikamentes, **dadurch gekennzeichnet, dass** mindestens ein nach einem der Ansprüche 1 bis 4 hergestelltes Polyglucan und / oder Polyglucanderivat als Tablettenhilfsstoff verwendet wird.

## Claims

1. A process for producing polyglucans and/or polyglucan derivatives, **characterised in that** at least one transferase and/or glycosyltransferase is added to amylosucrase *in vitro*.

2. A process according to claim 1, **characterised in that** the amylosucrase comprises an amino acid sequence according to SEQ ID. No. 1.

3. A process according to claim 1, **characterised in that** the polyglucan derivative is a polyglucan ester or a polyglucan ether.

4. A process according to claim 1, **characterised in that** the polyglucan derivative is a graft polymer, block polymer, copolymer, random copolymer or a star polymer, ladder polymer or ribbon polymer.

5. A process for producing a medicament, **characterised in that** at least one polyglucan and/or polyglucan derivative produced according to one of claims 1 to 4 is used as active ingredient carrier.

6. A process for producing a medicament, **characterised in that** at least one polyglucan and/or polyglucan derivative produced according to one of claims 1 to 4 is used as a depot system for at least one active ingredient having a therapeutic or prophylactic effect.

7. A process for producing a medicament, **characterised in that** at least one polyglucan and/or polyglucan derivative produced according to one of claims 1 to 4 is used as a tablet exciplent.

## Revendications

1. Procédé pour la fabrication de polyglucanes et/ou de dérivés de polyglucanes, **caractérisé en ce que**, l'amylosucrase est disloquée en vitro par au moins une transférase et/ou une glycoslltransférase,

2. Procédé selon la revendication 1, **caractérisé en ce que**, l'amylosucrase comprend une séquence d'aminoacides conforme à SEQ ID. No. 1.

3. Procédé selon la revendication 1, **caractérisé en ce que**, le dérivé de polyglucane, est un polyglucane ester ou un polyglucane éther.

4. Procédé selon la revendication 1, **caractérisé en ce que**, le dérivé de polyglucane, est un copolymère statistique bouchon, bloc, co-copolymère ou un polymère étoile, conducteur ou bande.

5. Procédé pour fabriquer un médicament, **caractérisé en ce qu'**au moins un polyglucane est un dérivé de polyglucane fabriqué selon l'une des revendications 1 à 4 et utilisé comme support pour une substance active.

6. Procédé pour fabriquer un médicament, **caractérisé en ce qu'**au moins un polyglucane est un dérivé de polyglucane fabriqué selon l'une des revendications 1 à 4 et utilisé comme système de dépôt pour au moins une substance active avec un effet thérapeutique ou prophylactique.

7. Procédé pour fabriquer un médicament, **caractérisé en ce qu'**au moins un polyglucane est un dérivé de polyglucane fabriqué selon l'une des revendications 1 à 4 et utilisé comme matière auxiliaire pour tablettes.


### SEQUENZPROTOKOLL

<110> Aventis Research & Technologies GmbH & Co KG
<120> Polyglucan und Polyglucanderivate erhältlich aus Amylosucrase biokatalytischer Herstellung und biogener Stoffe
<130> 98F115
<140> 198 46 492.4
   <141> 1998-10-09
<150> 198 46 492.4
   <151> 1998-10-09
<160> 1
<170> PatentIn Ver. 2.1
<210> 1
   <211> 636
   <212> PRT
   <213> Neisseria polysaccharea
<400> 1
   Met Leu Thr Pro Thr Gln Gln Val Gly Leu Ile Leu Gln Tyr Leu Lys
   1 5 10 15
   Thr Arg Ile Leu Asp Ile Tyr Thr Pro Glu Gln Arg Ala Gly Ile Glu
   20 25 30
   Lys Ser Glu Asp Trp Arg Gln Phe Ser Arg Arg Met Asp Thr His Phe
   35 40 45
   Pro Lys Leu Met Asn Glu Leu Asp Ser Val Tyr Gly Asn Asn Glu Ala
   50 55 60
   Leu Leu Pro Met Leu Glu Met Leu Leu Ala Gln Ala Trp Gln Ser Tyr
   65 70 75 80
   Ser Gln Arg Asn Ser Ser Leu Lys Asp Ile Asp Ile Ala Arg Glu Asn
   85 90 95
   Asn Pro Asp Trp Ile Leu Ser Asn Lys Gln Val Gly Gly Val Cys Tyr
   100 105 110
   Val Asp Leu Phe Ala Gly Asp Leu Lys Gly Leu Lys Asp Lys Ile Pro
   115 120 125
   Tyr Phe Gln Glu Leu Gly Leu Thr Tyr Leu His Leu Met Pro Leu Phe
   130 135 140
   Lys Cys Pro Glu Gly Lys Ser Asp Gly Gly Tyr Ala Val Ser Ser Tyr
   145 150 155 160
   Arg Asp Val Asn Pro Ala Leu Gly Thr Ile Gly Asp Leu Arg Glu Val
   165 170 175
   Ile Ala Ala Leu His Glu Ala Gly Ile Ser Ala Val Val Asp Phe Ile
   180 185 190
   Phe Asn His Thr Ser Asn Glu His Glu Trp Ala Gln Arg Cys Ala Ala
   195 200 205
   Gly Asp Pro Leu Phe Asp Asn Phe Tyr Tyr Ile Phe Pro Asp Arg Arg
   210 215 220
   Met Pro Asp Gln Tyr Asp Arg Thr Leu Arg Glu Ile Phe Pro Asp Gln
   225 230 235 240
   His Pro Gly Gly Phe Ser Gln Leu Glu Asp Gly Arg Trp Val Trp Thr
   245 250 255
   Thr Phe Asn Ser Phe Gln Trp Asp Leu Asn Tyr Ser Asn Pro Trp Val
   260 265 270
   Phe Arg Ala Met Ala Gly Glu Met Leu Phe Leu Ala Asn Leu Gly Val
   275 280 285
   Asp Ile Leu Arg Met Asp Ala Val Ala Phe Ile Trp Lys Gln Met Gly
   290 295 300
   Thr Ser Cys Glu Asn Leu Pro Gln Ala His Ala Leu Ile Arg Ala Phe
   305 310 315 320
   Asn Ala Val Met Arg Ile Ala Ala Pro Ala Val Phe Phe Lys Ser Glu
   325 330 335
   Ala Ile Val His Pro Asp Gln Val Val Gln Tyr Ile Gly Gln Asp Glu
   340 345 350
   Cys Gln Ile Gly Tyr Asn Pro Leu Gln Met Ala Leu Leu Trp Asn Thr
   355 360 365
   Leu Ala Thr Arg Glu Val Asn Leu Leu His Gln Ala Leu Thr Tyr Arg
   370 375 380
   His Asn Leu Pro Glu His Thr Ala Trp Val Asn Tyr Val Arg Ser His
   385 390 395 400
   Asp Asp Ile Gly Trp Thr Phe Ala Asp Glu Asp Ala Ala Tyr Leu Gly
   405 410 415
   Ile Ser Gly Tyr Asp His Arg Gln Phe Leu Asn Arg Phe Phe Val Asn
   420 425 430
   Arg Phe Asp Gly Ser Phe Ala Arg Gly Val Pro Phe Gln Tyr Asn Pro
   435 440 445
   Ser Thr Gly Asp Cys Arg Val Ser Gly Thr Ala Ala Ala Leu Val Gly
   450 455 460
   Leu Ala Gln Asp Asp Pro His Ala Val Asp Arg Ile Lys Leu Leu Tyr
   465 470 475 480
   Ser Ile Ala Leu Ser Thr Gly Gly Leu Pro Leu Ile Tyr Leu Gly Asp
   485 490 495
   Glu Val Gly Thr Leu Asn Asp Asp Asp Trp Ser Gln Asp Ser Asn Lys
   500 505 510
   Ser Asp Asp Ser Arg Trp Ala His Arg Pro Arg Tyr Asn Glu Ala Leu
   515 520 525
   Tyr Ala Gln Arg Asn Asp Pro Ser Thr Ala Ala Gly Gln Ile Tyr Gln
   530 535 540
   Gly Leu Arg His Met Ile Ala Val Arg Gln Ser Asn Pro Arg Phe Asp
   545 550 555 560
   Gly Gly Arg Leu Val Thr Phe Asn Thr Asn Asn Lys His Ile Ile Gly
   565 570 575
   Tyr Ile Arg Asn Asn Ala Leu Leu Ala Phe Gly Asn Phe Ser Glu Tyr
   580 585 590
   Pro Gln Thr Val Thr Ala His Thr Leu Gln Ala Met Pro Phe Lys Ala
   595 600 605
   His Asp Leu Ile Gly Gly Lys Thr Val Ser Leu Asn Gln Asp Leu Thr
   610 615 620
   Leu Gln Pro Tyr Gln Val Met Trp Leu Glu Ile Ala
   625 630 635